# EUROPEAN PATENT SPECIFICATION

(11) **EP 4 326 207 B1**
(45) Date of publication and mention of the grant of the patent: **31.12.2025**
(21) Application number: 22792268.9
(22) Date of filing: 18.04.2022
(51) Int. Cl.: A61F 13/15, A61F 13/20, A61F 5/44, A61F 5/455, A61F 13/24, A61F 6/08

(54) **MENSTRUAL DISC**
MENSTRUATIONSSCHEIBE
DISQUE MENSTRUEL

(30) Priority: 19.04.2021 US 202163176685 P; 15.10.2021 US 202163256092 P
(43) Date of publication of application: 28.02.2024
(73) Proprietor: LYV LIFE INC. DBA CORA, San Francisco, California 94107 (US)
(72) Inventor: BRUSH, Jennifer, Walnut Creek, California 94595 (US); SANDROLINI, Emma, Cupertino, California 95014 (US); METZ, Marla, San Diego, California 92120 (US); NEWMAN, Morgen, Mill Valley, California 94941 (US)
(74) Representative: Plougmann Vingtoft a/s
(86) International application number: PCT/US2022/025214
(87) International publication number: WO 2022/225848

(56) References cited:
- US-A- 3 371 664
- US-A- 4 703 752
- US-A- 5 771 900
- US-A1- 2016 278 988
- US-A1- 2018 214 298
- US-A1- 2019 282 350
- US-B1- 6 796 973
- 30 March 2021 (2021-03-30), Retrieved from the Internet <URL:https://www.amazon.com/Cora-Reusable-Sustainable-Alternative-Eco-Friendiy/dp/B091DDT562?ref_=ast_sto_dp&th=1>

## Description

### BACKGROUND

The invention generally relates to reusable feminine hygiene products and their use in managing menstruation, and more specifically, to features that improve the insertion and removal process, overall comfort/fit, and capacity/wear time to improve the period experience and reduce environmental waste.

Menstrual Discs are an emerging category in reusable/sustainable period care and still somewhat undefined. The reusable period category includes menstrual cups, menstrual discs, reusable pads and period underwear. In addition, there are disposable menstrual discs that fulfill slightly different user needs. Since the category is nascent, there are only a few reusable menstrual discs in market currently and most brands are sold DTC and/or outside of the USA. There is not an established "state of the art" norm in discs. The disposable discs (produced by Flex/Softdisc) have the longest history in the category, but are not reusable, so do not resonate as strongly with consumers seeking more sustainable period care products. A menstrual disc disclosing the features of the preamble of claim 1 is "Cora Menstrual Disc | Reusable Period Disc | Wear Up to 12-Hours | Sustainable Alternative to Tampons/Pads | for Light/Heavy Flows | Leak Proof | Medical Grade Silicone | Eco-Friendly Feminine Hygiene", Amazon.com, 30 March 2021 (2021-03-30), pages 1-9, XP093080160. URL:https://www.amazon.com/Cora-Reusable-Sustainable-Altemative-Eco-Friendly/dp/ B091DDT562?th=1

Menstrual cups are more prevalent and there are many brands in-market. However, menstrual cups are also still emerging. That said, menstrual cups have gained traction in the past 4 years and are an entry point to the reusable products category. Sizing is one of the main pain points in menstrual cups. There are two main factors in selecting the right size cup: pelvic floor strength and cervix height. Most women are unaware of their own pelvic floor strength and cervix height. Thus, a universally sized disc would solve the sizing problem- and potentially increase the adoption of reusable products.

Lumma, Nixit, and Intimina all have reusable menstrual discs. Because the disc sits higher up in the vagina (in the vaginal fornix), many women have trouble removing the disc-citing examples of messy removal and/or not being able to get a firm grip on the edge of the disc. Only Lumma has attempted to innovate removal with the inclusion of a removal string. In our research, some women find the string too long/uncomfortable. One of the benefits of a menstrual disc is that it can remain in place and allow a woman to have sexual intercourse during her period. Lumma's removal string interfered with this benefit.

Both Nixit and Intimina's products are on the larger range of diameter and intimidating to some women (for insertion). Only Lumma has attempted small, medium and large sizing of the menstrual disc to address female anatomy. The diaphragm has a different purpose than the menstrual disc (preventing pregnancy versus collecting menses) but anatomically, sits in the same place in a woman's body, in the vaginal fornix.

In testing Nixit and Intimina, insertion was difficult. Intimina's soft rim made the disc flexible and easy to fold-but once inside the vagina, it was very difficult to open in the vaginal fornix and orient properly. Nixit's firm rim was easier to guide into place, but their thicker catch made the product uncomfortable once inserted.

The present invention attempts to solve these problems as well as others.

### SUMMARY OF THE INVENTION

Provided herein are systems, methods and apparatuses for a Menstrual disc. In one embodiment, the Menstrual disc comprises an inner rim operably connected with a pull tab and a central catch portion, wherein the inner rim and the pull tab form a generally C-shape opening; the central catch portion operably connected with an outer rim including at least one grip portion and a curved top portion; wherein the outer rim is a semi-firm compliant rim that operates to fold inward towards an axis of the Menstrual disc to conform to a folded configuration from an open configuration; the axis runs from a front portion of the Menstrual disc to a back portion; the at least one grip portion is positioned perpendicular to the axis to permit folding of the Menstrual disc inwards towards the axis; and the central catch portion is operable to contain a menstrual fluid. In one embodiment, the menstrual disc minimizes a suctional force when temporarily installed in anatomical lumen.

A method of using a Menstrual disc comprises: folding the Menstrual disc to a folded configuration to a diameter D2 from a diameter D1 from an open configuration, wherein the diameter D2 of the folded configuration is between about 15mm and about 35mm; inserting the diameter D2 of the folded configuration through the vagina; tucking a front rim of the Menstrual disc up and behind the pubic bone to position the Menstrual disc; unfolding the Menstrual disc to the open configuration once the Menstrual disc is under the cervix; tucking a top rim portion of the Menstrual disc under the pubic bone, such that the Menstrual Disc sits within the fornix without a seal; and collecting menstrual fluid with the Menstrual disc during a menstrual period. In one embodiment, the method minimizes a suction force when the menstrual disc is temporarily installed within the fornix.

The methods, systems, and apparatuses are set forth in part in the description which follows, and in part will be obvious from the description, or can be learned by practice of the methods, apparatuses, and systems. The advantages of the methods, apparatuses, and systems will be realized and attained by means of the elements and combinations particularly pointed out in the appended claims. It is to be understood that both the foregoing general description and the following detailed description are exemplary and explanatory only and are not restrictive of the menstrual discs as claimed.

### BRIEF DESCRIPTION OF THE DRAWINGS

In the accompanying figures, like elements are identified by like reference numerals among the several preferred embodiments of the present invention.
**FIG. 1A** is a perspective view of the Menstrual disc, according to one embodiment; and **FIG. 1B** is a perspective view of the Menstrual disc for minimizing suction, according to one embodiment.
**FIG. 2A** is a side view of the Menstrual disc showing the grip portion, according to one embodiment; and **FIG. 2B** is a side view of the Menstrual disc showing the grip portion and the hole.
**FIG. 3A** is a top view of the Menstrual disc, according to one embodiment; and **FIG. 3B** is a top view of the Menstrual disc for minimizing suction, according to one embodiment.
**FIG. 4A** is a front view of the Menstrual disc showing the pull tab, according to one embodiment; and **FIG. 4B** is a front view of the Menstrual disc showing the pull tab and hole features, according to one embodiment.
**FIG. 5A** is a cross-sectional view of the Menstrual disc taken along lines 5-5 from **FIG. 4A****,** according to one embodiment; and **FIG. 5B** is a cross-sectional view of the Menstrual disc taken along lines 5-5 from **FIG. 4B****,** according to one embodiment.
**FIG. 6** is a bottom perspective view showing the Menstrual disc and the pull tab, according to one embodiment.
**FIG. 7A** is a back view of the Menstrual disc, according to one embodiment; and **FIG. 7B** is a back view of the Menstrual disc for minimizing suction, according to one embodiment.
**FIG. 8A** is a side view of the Menstrual disc showing the grip portion, according to one embodiment; **FIG. 8B** is a side view of the Menstrual disc for minimizing suction, according to one embodiment.
**FIG. 9A** is a cross-sectional view of the Menstrual disc taken along lines 9-9 from **FIG. 8A**, according to one embodiment; and **FIG. 9B** is a cross-sectional view of the Menstrual disc taken along lines 9-9 from **FIG. 8B**, according to one embodiment.
**FIG. 10** is a bottom view of the Menstrual disc showing the pull tab, according to one embodiment.
**FIG. 11** is a depiction of the Menstrual disc disposed in the folded configuration, according to one embodiment.
**FIG. 12** is a schematic display of the cervix and fornix anatomy where the Menstrual disc is operably disposed.

### DETAILED DESCRIPTION OF THE INVENTION

The foregoing and other features and advantages of the invention are apparent from the following detailed description of exemplary embodiments, read in conjunction with the accompanying drawings. The detailed description and drawings are merely illustrative of the invention rather than limiting, the scope of the invention being defined by the appended claims.

Embodiments of the invention will now be described with reference to the Figures, wherein like numerals reflect like elements throughout. The terminology used in the description presented herein is not intended to be interpreted in any limited or restrictive way, simply because it is being utilized in conjunction with detailed description of certain specific embodiments of the invention. Furthermore, embodiments of the invention may include several novel features, no single one of which is solely responsible for its desirable attributes or which is essential to practicing the invention described herein.

The use of the terms "a" and "an" and "the" and similar referents in the context of describing the invention are to be construed to cover both the singular and the plural, unless otherwise indicated herein or clearly contradicted by context. It will be further understood that the terms "comprises," "comprising," "includes," and/or "including," when used herein, specify the presence of stated features, integers, steps, operations, elements, and/or components, but do not preclude the presence or addition of one or more other features, integers, steps, operations, elements, components, and/or groups thereof.

Recitation of ranges of values herein are merely intended to serve as a shorthand method of referring individually to each separate value falling within the range, unless otherwise indicated herein, and each separate value is incorporated into the specification as if it were individually recited herein. The word "about," when accompanying a numerical value, is to be construed as indicating a deviation of up to and inclusive of 10% from the stated numerical value. The use of any and all examples, or exemplary language ("e.g." or "such as") provided herein, is intended merely to better illuminate the invention and does not pose a limitation on the scope of the invention unless otherwise claimed. No language in the specification should be construed as indicating any nonclaimed element as essential to the practice of the invention.

References to "one embodiment," "an embodiment," "example embodiment," "various embodiments," etc., may indicate that the embodiment(s) of the invention so described may include a particular feature, structure, or characteristic, but not every embodiment necessarily includes the particular feature, structure, or characteristic. Further, repeated use of the phrase "in one embodiment," or "in an exemplary embodiment," do not necessarily refer to the same embodiment, although they may.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts. Unless otherwise expressly stated, it is in no way intended that any method or aspect set forth herein be construed as requiring that its steps be performed in a specific order. Accordingly, where a method claim does not specifically state in the claims or descriptions that the steps are to be limited to a specific order, it is no way intended that an order be inferred, in any respect. This holds for any possible non-express basis for interpretation, including matters of logic with respect to arrangement of steps or operational flow, plain meaning derived from grammatical organization or punctuation, or the number or type of aspects described in the specification.

Generally speaking, the Menstrual disc is a reusable feminine hygiene product and the method of using the Menstrual disc is for managing menstruation, and more specifically, to features that improve the insertion and removal process, overall comfort/fit, and capacity/wear time to improve the period experience and reduce environmental waste.

The Menstrual disc is a simple, reusable alternative to pads and tampons designed to fit most women and stay in place comfortably. The Menstrual disc comprises a finger-width groove on the underside of the rim with a textured surface to improve grip, wherein the finger-width groove allows a user to easily hook their finger on the underside on the disc. This finger-width groove provides a method for emptying the Menstrual disc during wear without removal of the Menstrual disc from the vagina. Alternatively, the method provides for the emptying the Menstrual disc with removal of the Menstrual disc from the vagina, and reuse after cleaning and sanitizing. More specifically, the menstrual disc relies on anatomical positioning, rather than suction, to stay in place and includes modifications to the rim to minimize a suction force of the menstrual disc such that the menstrual disc provides the user with protection from leakage of fluid collected in the menstrual disc, and allows the menstrual disc to be easily removed from an anatomical lumen. In one embodiment, the suction force is minimized by at least 5 N, alternatively, between about 5 and 10 N, alternatively at least about 15 N. In one embodiment, the menstrual disc creases the suction force or vacuum over a period of time. The period of time is between about 1 hour and about 24 hours, alternatively, between about 1 hour and about 48 hours, alternatively at least 1 hour. The menstrual disc minimizes the pressure on the fluid collection side as to allow the removal of the menstrual disc in a fashion requiring less force due to a vacuum being minimized.

In one embodiment, the menstrual disc minimizes the suction force of the menstrual disc once temporarily installed into the fornix by providing a plurality of holes near or in the rim of the menstrual disc, or in another part of the menstrual disc, that allow some amount of air to pass from the inside of the disc to the outside, and from the outside of the disc to the inside. In one embodiment, the menstrual disc comprises a rim that is not flat, such that the rim includes an exterior surface that is more difficult to suction when the menstrual disc is temporarily installed in the vaginal canal. In one embodiment, the menstrual disc includes a texture or a patterned surface on the exterior of the rim that helps break suction of the menstrual disc when temporarily installed in the vaginal canal. In one embodiment, the menstrual disc comprises a thinner membrane or layer in the catch that does create as much pressure on the outer rim as to create suction of the menstrual disc when temporarily installed in the fornix.

The Menstrual disc comprises a diameter and a rim height specifically selected based on the anatomical features of the female body. Pelvic floor strength (influenced by age, childbirth, and weight, among other factors), cervix height (varies during the menstrual cycle) and fluid capacity are all size, property, and dimension considerations for the Menstrual disc. The diameter contribute to proper fit/comfort and universal sizing for most women, while the rim height and diameter are related to the capacity of the Menstrual disc. The diameter and rim height provide a fluid capacity to hold between about 30 ml to about 50 ml, alternatively about 47.5 mL of menstrual fluid for a comparatively long wear time up to about 12 hours. The Menstrual disc comprises the rim height with a thin catch, a side rim finger grip that provides the method of the folding and inserting the Menstrual disc through the vagina toward and contacting the cervix, and disposing the Menstrual disc within the fornix.

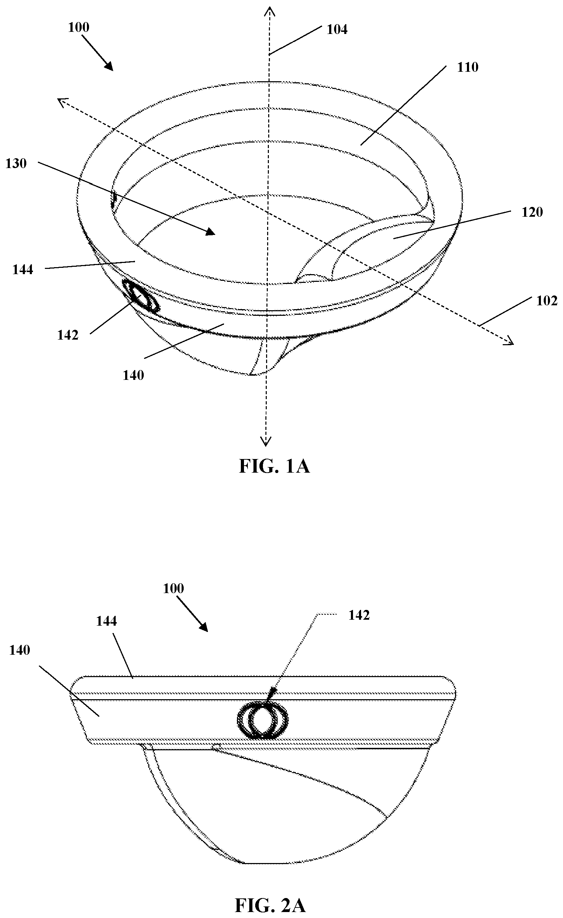

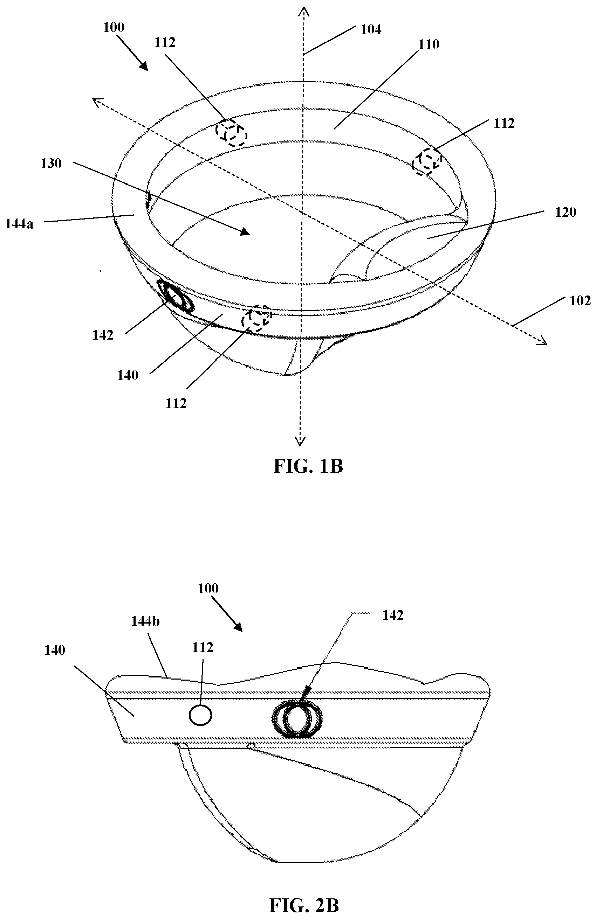

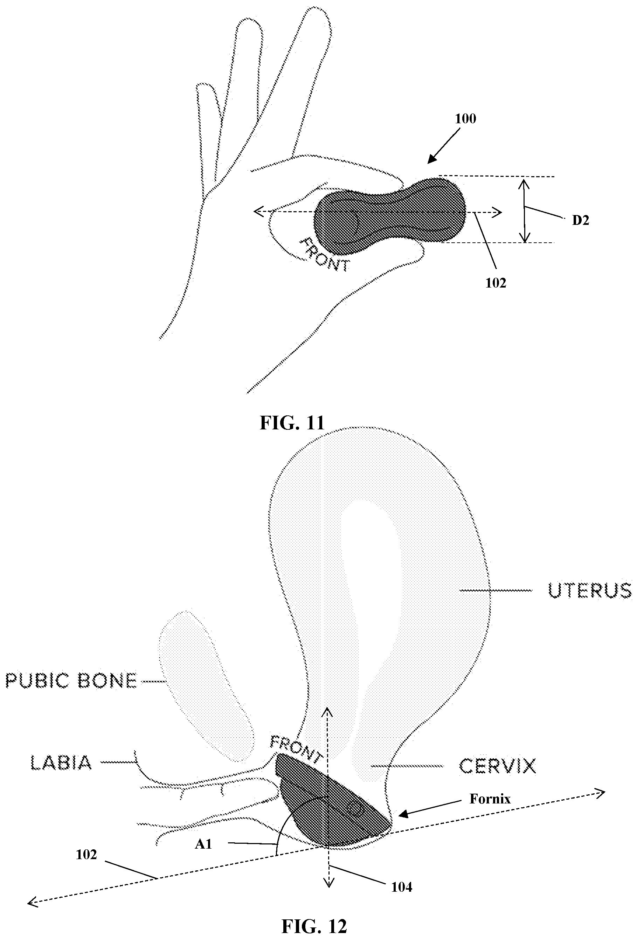

As shown in **FIGS. 1A-1B**, the Menstrual disc 100 is a generally circular configuration and comprises an inner rim 110 operably connected with a pull tab 120 and a central catch portion 130. The central catch portion 130 is operably connected with an outer rim 140 including at least one grip portion 142 and a curved top portion 144a, as shown in **FIG. 2A**, or, as shown in **FIG. 2B**, a curvilinear top portion 144b provides an undulating top surface to minimize the suction force on the top of the menstrual disc 100 when temporarily implanted on the fornix. The outer rim 140 is a semi-firm compliant rim or semi-resilient or semi-elastic rim that operates to fold inward towards an axis 102 of the Menstrual disc to conform to a folded configuration from an open configuration, as shown in **FIGS. 1A-1B****.** The axis 102 runs from a front portion of the Menstrual disc 100 to a back portion and will substantially align with the sagittal plane of the human body when the Menstrual disc 100 is removably implanted or temporarily installed within vagina. The at least one grip portion 142 allows a user to fold the Menstrual disc along the axis 102 to the folded configuration and the grip portion 142 minimizes slippage during folding and insertion. The at least one grip portion 142 is positioned perpendicular to the axis 102 to permit folding of the Menstrual disc inwards towards axis 102. The at least one grip portion 142 includes a frictional component to allow a user to grip and fold the Menstrual disc into the folded configuration and insert the Menstrual disc without the user losing grip on the Menstrual disc. The folded configuration is operable for a user to insert the Menstrual disc and the open configuration is operable to cover the cervix within the fornix. The cervix projects into the vagina, and the circular trough formed at the upper end of the vagina around the cervix is the fornix. When removably implanted in the open configuration, the Menstrual disc 100 sits at an angle A1 off its longitudinal axis 104, as shown in **FIG. 12**. The central catch portion 130 is operable to contain, catch, or maintain menstrual fluid during a menstrual period and optimizes comfort for the user. As shown in **FIGS. 1B, 2B****,** and **5B****,** the inner rim 110 and the outer rim 140 includes a plurality of holes 112 disposed through the thickness of the inner rim 110 to the outer rim 140, as to provide suction relief on the side of the outer rim 140 of the menstrual disc 100 when temporarily installed in the vaginal wall. The plurality of holes 112 may include at least one hole 112 or between about one hole and about 5 holes depending on the amount suction needed to be prevented. The top portion of the pull tab 120 is operably connected to the top portion of the outer rim 140 to generate a non-annular opening or a generally C-shape opening of the Menstrual disc 100 in the open configuration.

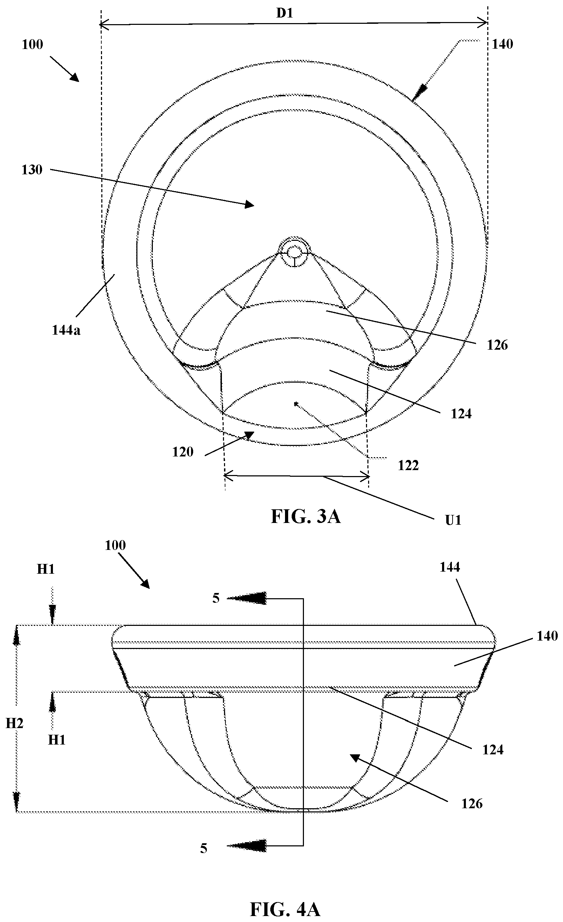

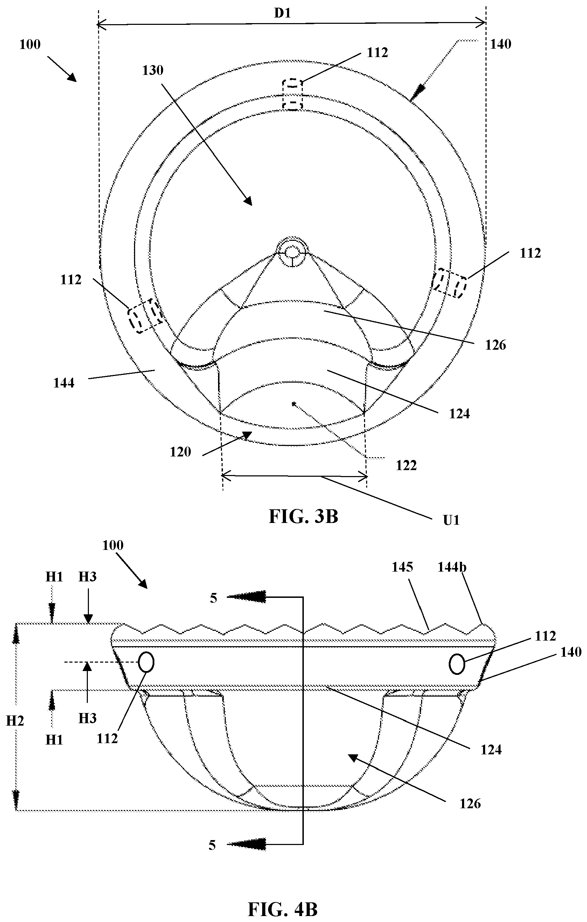

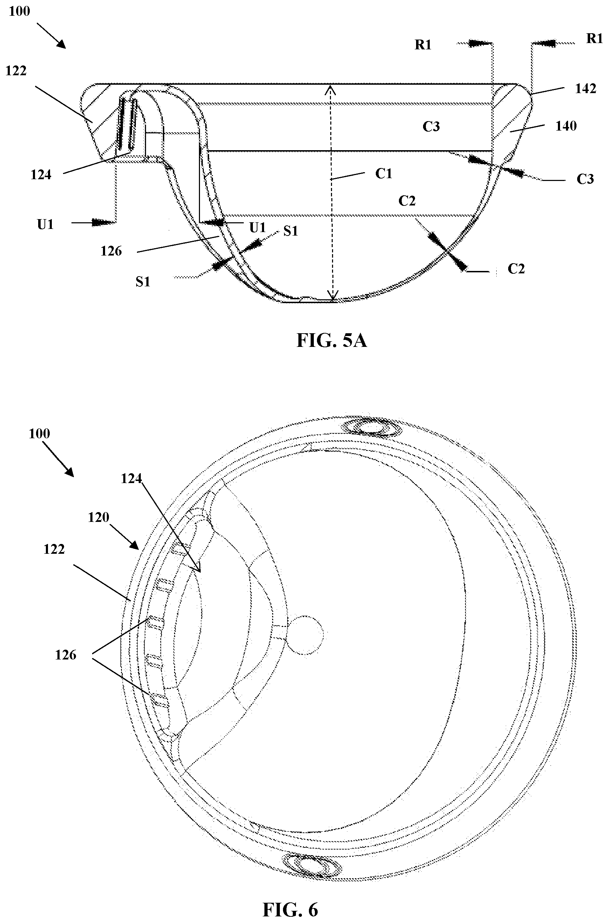

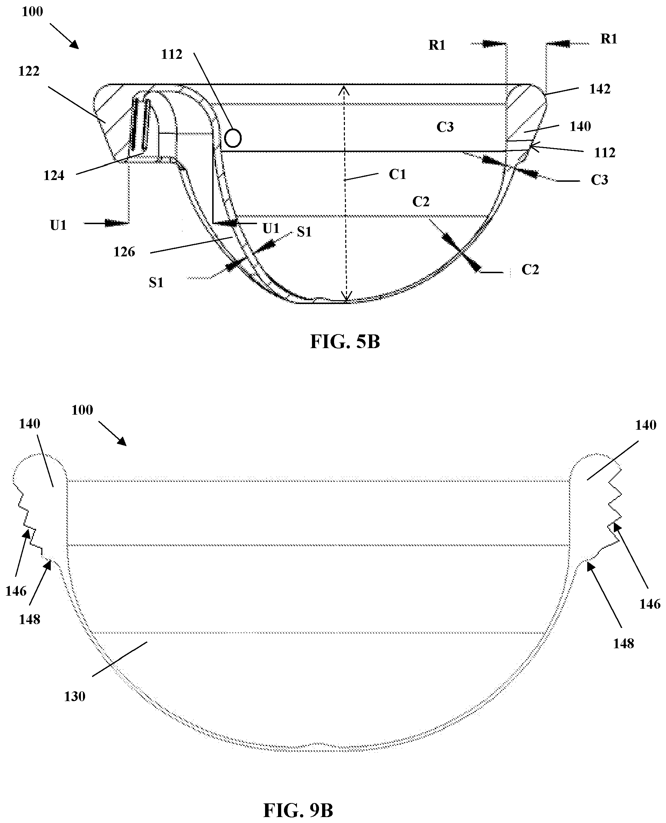

As shown in **FIG. 3A-3B**, the Menstrual disc 100 and the curved top portion 144a, 144b includes a diameter D1, the upper rim 110 includes a rim height H1, and the Menstrual disc 100 includes a disc height H2, as shown in **FIGS. 4A-4B****.** As shown in FIG. 3B, the plurality of holes 112 are positioned around the circumference of the outer rim 140, and in one embodiment, the plurality of holes 112 are positioned equidistantly from each other. If three holes 112 are deployed for minimizing suction, then the holes 112 are positioned about 120 degrees in separation from the adjacent hole 112. In one embodiment, the curved top portion 144b includes a textured surface 145, as shown in **FIG. 4B**. The textured surface 145 prevents suction at force F1 or provides suction relief on the top portion of the menstrual disc 100. The plurality of holes are positioned at a height H3 from the top of the outer rim 140. In one embodiment, the height H3 is between about 6mm and about 10mm, alternatively, between about 7mm and about 9mm, alternatively, about 8mm. In one embodiment, the diameter D1 is between about 60mm and 70mm, alternatively, between about 62mm and about 68mm, alternatively between about 64mm and about 66mm, alternatively about 65mm. In one embodiment, the rim height H1 is between about 9mm and about 14mm, alternatively, between about 10mm and about 13mm, alternatively, between about 11mm and about 12mm, alternatively about 11mm. The disc height H2 is between about 28mm and about 35mm, alternatively, between about 29mm and about 34mm, alternatively, between about 30mm and 33mm, alternatively, about 31mm. The curved top portion 144 includes a radius of curvature, as shown in **FIGS. 5A-5B****.** The radius of curvature between about 4mm and about 8 mm, alternatively, between about 5mm and about 7mm, alternatively, about 6mm. The radius of curvature is sized to permit smooth insertion of the Menstrual disc through the vagina opening and the cervix.

The upper rim 110 and the outer rim 140 include a rim thickness R1, as shown in **FIGS. 5A-5B**. In one embodiment, R1 is between about 5.0mm and about 6.0mm, alternatively, between about 5.1mm and about 5.9mm, alternatively, between about 5.2mm and about 5.8mm, alternatively about 5.75mm. In one embodiment, the ratio of the rim height H1 and rim thickness R1 is between about 0.9 and about 2.9, alternatively the ratio of H1:R1 is between about 1.2 and about 2.7, alternatively, between about 1.5 and about 2.5, alternatively, between about 1.7 and about 2.2, alternatively, about 1.83, alternatively, about 1.91. In one embodiment, the ratio of rim height H1 to rim thickness R1 is less than 2. The ratio of rim height H1 to rim thickness R1 is sized to provide optimal folding capability of the Menstrual Disc while permitting the rim to sit against the fornix during operation. The rim height H1 relates to the capacity of the disc; the rim thickness R1 relates to the firmness, which allows for an easier insertion experience and allows the user to get the disc in place more easily.

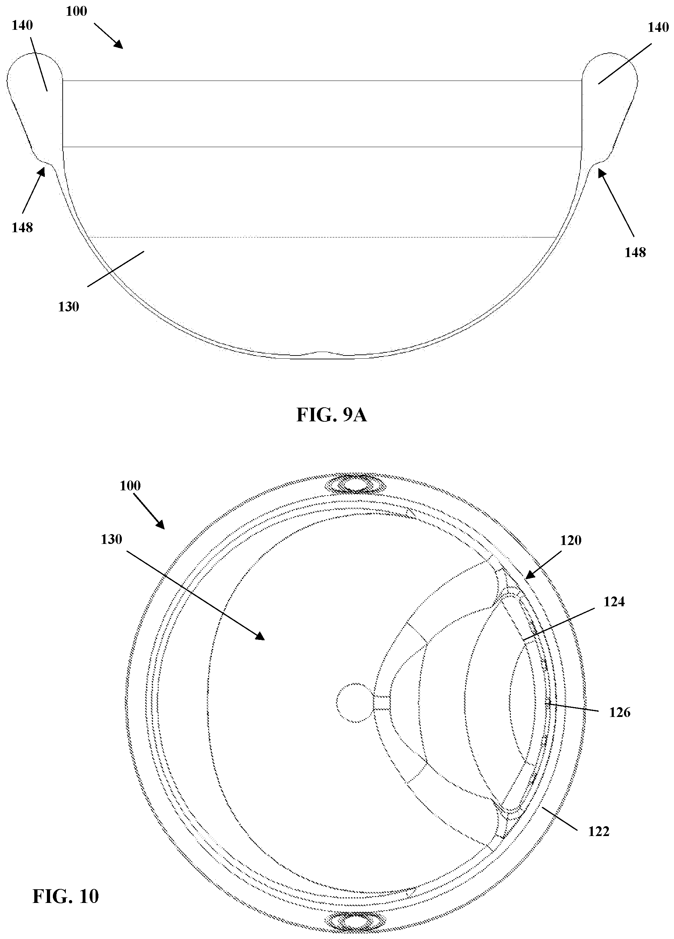

The pull tab 120 includes a rim portion 122, an underside groove 124, and a sloped portion 126, as shown in **FIGS. 3A**-**3B**. The rim portion 122 operably couples with the curved top portion 144 of the outer rim 140 to create the non-annular or generally C-shape opening of the Menstrual Disc 100. The rim portion 122 of the pull tab 120 provides additional structural support for the outer rim 140 when the Menstrual disc is in the open configuration and temporarily implanted. The underside groove 124 includes a width U1, as shown in **FIGS. 3A-3B****,** and a depth U2, as shown in **FIGS. 5A-5B****.** The width U1 and the depth U2 is a groove sized to permit a user to locate the Menstrual disc within the vagina and sized for the user's finger to grip the pull tab 120 and withdraw the Menstrual disc from the vagina. The width U1 and the depth U2 is also the approximate width and depth of the rim portion 122 of the pull tab 122. The width and depth of the rim portion 122 contributes to the structural support of the Menstrual disc in the open configuration when temporarily implanted. In one embodiment, the underside groove 124 includes a textured surface 126, as shown in **FIGS. 6** and **10**, to permit a user's fingertip to grip the pull tab 120 sufficiently within the vagina. The textured surface 126 includes a plurality of tabs designed with a raised geometric component to permit a user to grip the underside groove 124 when wet or covered by a fluid or menstrual fluid. In one embodiment, the raised geometric component includes a polygonal or quadrilateral profile. The pull tab 120 is oriented closest to the vaginal opening and the rim portion 122 along with the underside groove 124 allows the user to tilt the Menstrual disc in an upright position to avoid spilling menstrual fluid during removal of the Menstrual disc from the vagina. In one embodiment, width U1 and depth U2 are between about 9mm and about 15mm, alternatively, between about 10mm and about 14mm, alternatively about 11m. The sloped portion 126 includes a width S1 between about 0.5 mm and about 1.5mm, alternatively, between about 0.7mm and about 1.2mm, alternatively between about 0.9 and 1.1mm. The sloped portion 126 is sized to operably connect with the central catch portion 130. The width S1 is sized to be greater than the thickness of the central catch portion 130, to permit greater stiffness when the pull tab 120 is pulled away from an axis of the Menstrual disc and allow menstrual fluid disposed in the central catch portion 130 to be disposed from the Menstrual disc when the pull tab 120 is engaged by a user. The rim portion 122 includes a triangular thickness profile, as shown in **FIGS. 5A-5B****.** The triangular thickness profile allows for the semi-rim stiffness of the outer rim 140 on the top portion of the Menstrual disc, while allowing for a thinner thickness on the bottom portion of the rim portion 122 to permit a user to deflect or grab the rim portion 122 of the pull tab 120. Each of the pull tab 120, underside groove 124, or textured surface 126 may be independently modified, sited, or shaped differently to meet the needs of the user, menstrual fluid, or anatomy.

As shown in **FIGS. 5A-5B**, the central catch portion 130 includes a catch height C1 and a catch capacity. In one embodiment, the catch capacity is between about 35 ml and about 50ml, alternatively, between about 46ml and 48ml, alternatively, about 47.5ml. The central catch portion 130 sits flat within the vaginal canal. And the catch capacity is the volume of a cylinder (Π r^2h). The volume of a cylinder calculation is used for the catch capacity of the Menstrual disc when not fully open once inserted into the vagina. The central catch portion 130 includes a catch thickness C2, as shown in **FIGS. 5A-5B**. The catch thickness C2 is sized to permit the central catch portion 130 to rest comfortably within the vagina, allow the Menstrual disc to fold to the folded configuration, and secure when menstrual fluid is disposed within the central catch portion 130. In one embodiment, the catch thickness C2 is between about 0.1mm and about 0.7mm, alternatively, between about 0.2mm and about 0.6mm, alternatively, between about 0.3mm and about 0.5mm, alternatively, about 0.4mm. In one embodiment, the catch thickness C2 is between about 0.01mm and about 0.20mm to provide a thinner catch portion 130 and prevent the pressure from building when the catch portion 130 includes a fluid as to prevent the menstrual disc from creating suction when temporarily installed in the vaginal canal. The catch thickness C2 expands to a rim thickness C3 when the central catch portion 130 connects with the outer rim 140. In one embodiment, C3 is between about 0.8mm and about 1.2mm, alternatively, between about 0.9mm and 1.1mm, alternatively about 1.0mm.

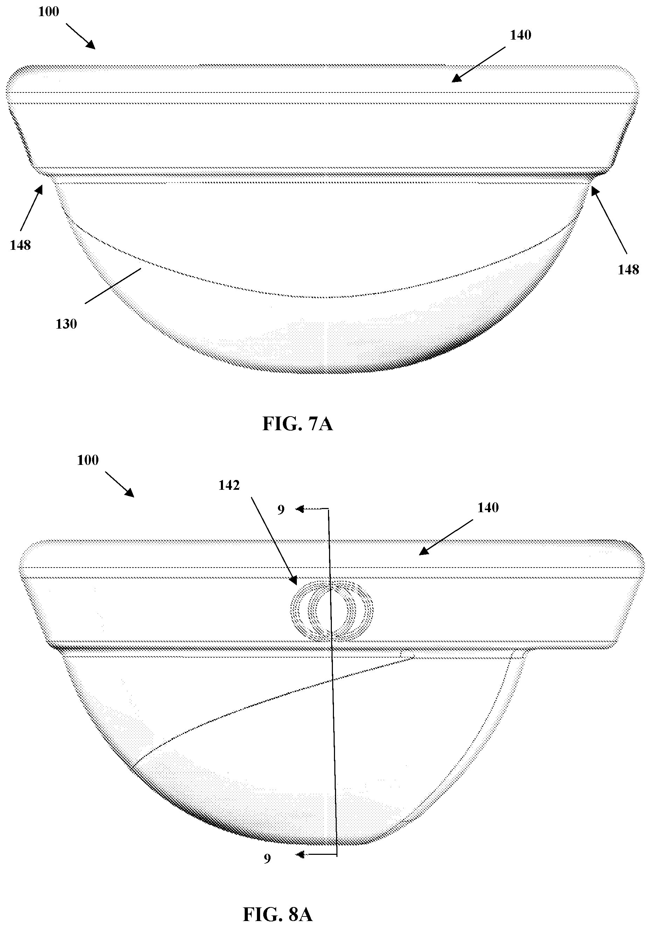

**FIGS. 8A-8B** is a side view of the Menstrual disc 100 showing the grip portion or textured portion 142, according to one embodiment. The grip portion 142 is comprised of at least two concentric circles that may be indentations on the outer rim 140 or raised portions on the outer rim 140 to provide the gripping action to flex the Menstrual disc to the folded configuration. In one embodiment, the textured portion 142 provides a textured surface that breaks a seal or suction of the menstrual disc when temporarily implanted. The indentations or raised portions of the textured portion 142 may be placed along the circumference of the outer rim 140. The at least two concentric circles may be overlapping or may be separated by a distance. The at least two concentric circles may include smaller concentric circles within a larger concentric circle to provide additional gripping portions. The diameter of the concentric circles may include a diameter approximating the tip of finger. In one embodiment, the concentric circles include at least 2 smaller concentric circles confined with a larger concentric circle.

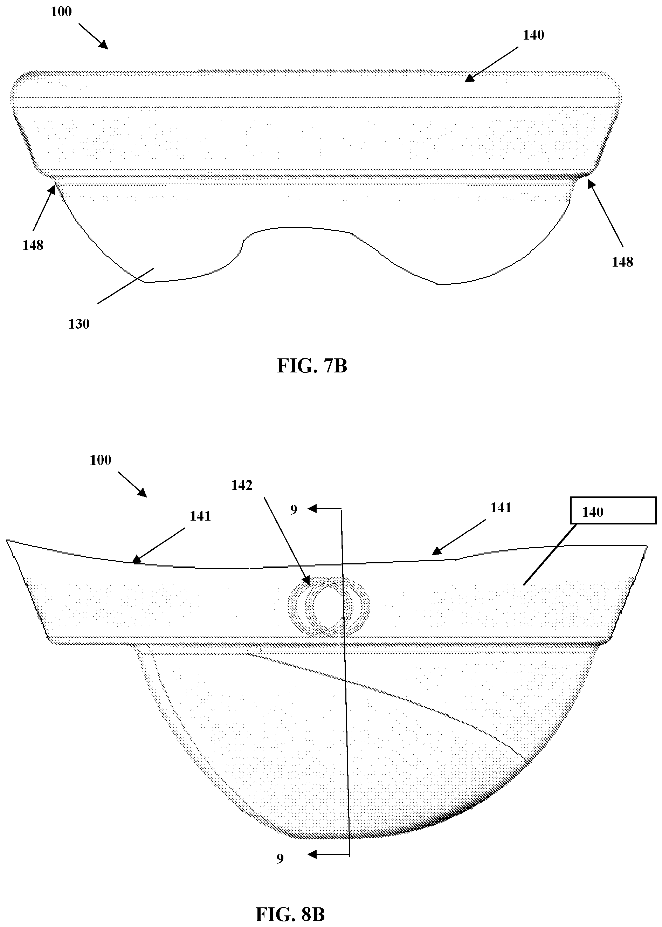

**FIGS. 7A-7B** and **9A-9B** show that the outer rim 140 comprises an outer lip 148 disposed around the outer surface of the outer rim 140 and between the outer surface of the central catch 130. The outer lip 148 provides a seated surface in which to dispose the Menstrual disc on the fornix. The outer lip 148 may include a seated surface between about 0.1mm and about 3.0 mm, alternatively between about 0.5mm and 2.5mm, alternatively, between about 0.9mm and 2.0mm. The semi-firm compliance of the outer rim 140 ensures the outer lip 148 and the seated surface maintain appropriate stiffness when the Menstrual disc is disposed on the fornix and when the central catch 130 includes menstrual fluid disposed therein. As shown in **FIG. 7B**, in one embodiment, the central catch 130 includes a smaller catch portion including an undulating cross-sectional profile to provide for minimizing the suction force of the menstrual disc 100. In one embodiment, the outer rim 140 includes a texture surface 146 that breaks any seal from forming with the vaginal wall, as shown in **FIG. 9B**. The textured surface 146 connects with the outer lip 148. As shown in **FIG. 8B**, the menstrual disc 100 includes a concave top portion 141 of the outer rim 140 to minimize the suction force of the menstrual disc 100 when temporarily installed on the fornix.

In operation, the Menstrual disc is folded to the folded configuration and includes a diameter D2, as shown in **FIG. 11**. In one embodiment, the folded configuration is a generally figure 8 configuration. The diameter D2 of the folded configuration is between about 15mm and about 35mm, alternatively, between about 20mm and about 30mm. The diameter D2 of the folded configuration permits the Menstrual disc to be inserted through the vagina, as shown in **FIG. 12**. The user guides the back rim of the Menstrual disc into the vagina and tucks the front rim up and behind the pubic bone to position the Menstrual disc. Once the user is under the cervix, the Menstrual disc is unfolded to the open, semi-hemispheric configuration, where the top rim portion tucks under the pubic bone and sits in the fornix without requiring suction. Cervix height is the distance from the vaginal opening to the cervix and the cervix height is on average between about 76.2mm and about 101.6mm (3 and about 4 inches) and varies to less than 76.2mm and up to about 127.0 mm (3 and up to about 5 inches). In one embodiment, the Menstrual disc sits higher in the vaginal canal and rests within the fornix to receive or catch menstrual fluid during a menstrual period. When removably implanted or temporarily installed in the open configuration, the Menstrual disc 100 sits at an angle A1 off its longitudinal axis 104 and the axis 102 which sits on the sagittal plane at the base of posterior cervix, as shown in **FIG. 12**. In one embodiment, the open configuration of the Menstrual disc when removably implanted is a smaller open configuration due to the top rim portion being bent inward. The Menstrual disc may collect fluid for an extended period of time up to about 12 hours when disposed on the fornix. The Menstrual disc may empty a fluid while the Menstrual disc is disposed within the fornix by the user gripping the pull tab and tilting the pull tab downwards towards the vaginal opening to remove the fluid. The underside groove of the pull tab aids in the removal process by allowing a user to easily hook their finger on the underside on the Menstrual disc and the user gently slides the Menstrual disc out of the vagina.

In one embodiment, the Menstrual disc is made from medical grade silicone, polymer, rubber, or other elastomeric or biocompatible material. The menstrual disc is intended to be washable and reusable.

In an alternative embodiment, the Menstrual disc may be used for stress incontinence. The placement of the Menstrual disc inside the vagina puts gentle pressure on the bladder and could function similar to a pessary. A pessary is a prosthetic device that can be inserted into the vagina to support its internal structure. It's often used in the case of urinary incontinence and a vaginal or pelvic organ prolapse. The Menstrual disc could catch urine or other fluids. The Menstrual disc may also be used as a specimen collector to collect blood and/or vaginal, cervical and/or uterine discharge, including for diagnostic purposes.

The Menstrual disc is not limited to the preferred embodiments described herein. For example, the Menstrual disc is not restricted to human use. The Menstrual disc may be used to collect discharge from non-human primates and other animals, and/or for substance delivery for veterinary applications. For non-human primate and other veterinary uses, the dimensions of the devices would be sized or adapted to fit the dimensions of the vaginal canal of the animal concerned.

While the invention has been described in connection with various embodiments, it will be understood that the invention is capable of further modifications. This application is intended to cover any variations, uses or adaptations of the invention following, in general, the principles of the invention, and including such departures from the present disclosure as, within the known and customary practice within the art to which the invention pertains.

## Claims

1. A menstrual disc (100), comprising: an inner rim (110) operably connected with a pull tab (120) and a central catch portion (130), wherein the inner rim 5 (110) and the pull tab (120) form a generally C-shape opening; the central catch portion (130) operably connected with an outer rim (140) including at least one grip portion (142) and a curved top portion (144a); wherein the outer rim (140) is a semi-firm and compliant rim that is configured to operate to fold inward towards an axis of the menstrual disc to conform to a folded configuration from an open 10 configuration; the axis runs from a front portion of the menstrual disc to a back portion; the at least one grip portion (142) is positioned perpendicular to the axis to permit folding of the menstrual disc inwards towards the axis; the central catch portion (130) is configured to be operable to contain a menstrual fluid in the open configuration and the folded configuration; wherein the pull tab (120) includes an 15 underside groove (124), a rim portion (122) and a sloped portion (126) extending downward from the inner rim (110) to the central catch portion (130); the rim portion (122) operably couples with the curved top portion (144a) of the outer rim (140) to create the generally C-shape opening; the underside groove (124) includes a width U1 and a depth U2, wherein the combination of the underside 20 groove (124), the inner rim (110) and a portion of the outer rim (140) are sized to be configured to allow a user to grip two or more of the pull tab (120), the inner rim (110) and the outer rim (140), to withdraw the menstrual disc from the vagina; wherein the width U1 and the depth U2 of the underside groove (124) is sized to permit a user to locate the Menstrual disc within the vagina and sized for the user's finger to grip the pull tab (120), and wherein the top of the pull tab (120) does not extend above the top of the outer rim (140) and the inner rim (110);
**characterized in that** the sloped portion (126) includes a width S1 sized to be greater than the thickness of the central catch portion (130), to permit greater stiffness when the pull tab (120) is pulled away from an axis of the Menstrual 30 disc; and **in that** the outer rim (140) includes a rim thickness R1 and a rim height H1, and the ratio of the rim height H1 and rim thickness R1 is between about 0.9 and about 2.0; the rim portion (122) includes a triangular thickness profile, which allows the outer rim (140) to include a semi-rim stiffness on the top and a thinner thickness on a bottom portion of the rim portion (122).

2. The menstrual disc of claim 1, the inner rim (110) and the outer rim (140) includes a plurality of holes (112) disposed through the thickness of the inner rim (110) to the outer rim (140), as to provide suction relief on the side of the outer rim (140) of the menstrual disc when temporarily installed in the vaginal wall.

3. The menstrual disc of claim 1, wherein the ratio of the rim height H1 and rim thickness R1 is about 1.83.

4. The menstrual disc of claim 2, wherein the thickness R1 is between about 5.0 mm and about 6.0 mm.

5. The menstrual disc of claim 3, wherein the menstrual disc includes a diameter D1, and the menstrual disc includes a disc height H2, wherein the diameter D1 is between about 60 mm and 70 mm, the rim height H1 is between about 9 mm and about 14 mm, and the disc height H2 is between about 28 mm and about 35 mm.

6. The menstrual disc of claim 1, wherein the central catch portion includes an undulating cross-sectional profile to provide for minimizing the suction force of the menstrual disc.

7. The menstrual disc of claim 6, wherein the outer rim (140) includes a textured surface (146) that breaks any seal from forming with the vaginal wall.

8. The menstrual disc of claim 6, wherein the width U1 and the depth U2 are between about 9 mm and about 15 mm; the sloped portion includes a width S1 between about 0.5 mm and about 1.5 mm, the width S1 is greater than a thickness of the central catch portion (130).

9. The menstrual disc of claim 1, wherein the central catch portion includes a catch height C1 and a catch capacity; wherein the catch capacity is between about 35 ml and about 50 ml when the central catch portion (130) sits within the vaginal canal; and the central catch portion (130) includes a catch thickness C2 between about 0.1 mm and about 0.7 mm; the catch thickness C2 expands to a rim thickness C3 when the central catch portion (130) connects with the outer rim (140); wherein the rim thickness C3 is between about 0.8 mm and about 1.2 mm.

10. The menstrual disc of claim 1, wherein the grip portion (142) comprises at least two concentric circles.

11. The menstrual disc of claim 10, wherein the outer rim (140) comprises an outer lip (148) disposed around the outer surface of the outer rim (140) and between the outer surface of the central catch.

## Patentansprüche

1. Menstruationsscheibe (100), umfassend: einen inneren Rand (110), der funktionsfähig mit einer Zuglasche (120) und einem zentralen Rastabschnitt (130) verbunden ist, wobei der innere Rand (110) und die Zuglasche (120) eine im Allgemeinen C-förmige Öffnung bilden; wobei der zentrale Rastabschnitt (130) funktionsfähig mit einem äußeren Rand (140) verbunden ist, der mindestens einen Griffabschnitt (142) und einen gekrümmten oberen Abschnitt (144a) einschließt; wobei der äußere Rand (140) ein halbfester und nachgiebiger Rand ist, der konfiguriert ist, um so zu arbeiten, dass er sich nach innen in Richtung einer Achse der Menstruationsscheibe faltet, um sich einer gefalteten Konfiguration aus einer offenen Konfiguration anzupassen; die Achse von einem vorderen Abschnitt der Menstruationsscheibe zu einem hinteren Abschnitt verläuft; der mindestens eine Griffabschnitt (142) senkrecht zur Achse positioniert ist, um das Falten der Menstruationsscheibe nach innen zur Achse hin zu ermöglichen; der zentrale Rastabschnitt (130) konfiguriert ist, um eine Menstruationsflüssigkeit in der offenen Konfiguration und der gefalteten Konfiguration aufnehmen zu können; wobei die Zuglasche (120) eine Unterseitenrille (124), einen Randabschnitt (122) und einen schrägen Abschnitt (126) einschließt, der sich von dem inneren Rand (110) nach unten zu dem zentralen Rastabschnitt (130) erstreckt; der Randabschnitt (122) funktionsfähig mit dem gekrümmten oberen Abschnitt (144a) des äußeren Randes (140) koppelt, um die Öffnung im Allgemeinen in C-Form zu erzeugen; die Unterseitenrille (124) eine Breite U1 und eine Tiefe U2 einschließt, wobei die Kombination aus der Unterseitenrille (124), dem inneren Rand (110) und einem Abschnitt des äußeren Randes (140) so bemessen ist, dass sie konfiguriert ist, um es einem Benutzer zu ermöglichen, zwei oder mehr der Zuglasche (120), des inneren Randes (110) und des äußeren Randes (140) zu ergreifen, um die Menstruationsscheibe aus der Vagina zu ziehen; wobei die Breite U1 und die Tiefe U2 der Unterseitenrille (124) so bemessen sind, dass ein Benutzer die Menstruationsscheibe in der Vagina platzieren kann, und so bemessen sind, dass der Finger des Benutzers die Zuglasche (120) greifen kann, und wobei die Oberseite der Zuglasche (120) nicht über die Oberseite des äußeren Randes (140) und des inneren Randes (110) hinausragt;
**dadurch gekennzeichnet, dass** der abgeschrägte Abschnitt (126) eine Breite S1 einschließt, die so bemessen ist, dass sie größer ist als die Dicke des zentralen Rastabschnitts (130), um eine größere Steifigkeit zu ermöglichen, wenn die Zuglasche (120) von einer Achse der Menstruationsscheibe weggezogen wird; und dass der äußere Rand (140) eine Randdicke R1 und eine Randhöhe H1 einschließt und das Verhältnis zwischen der Randhöhe H1 und der Randdicke R1 zwischen etwa 0,9 und etwa 2,0 beträgt; der Randabschnitt (122) ein dreieckiges Dickenprofil einschließt, das es dem äußeren Rand (140) ermöglicht, eine Halbrandsteifigkeit an der Oberseite und eine dünnere Dicke an einem unteren Abschnitt des Randabschnitts (122) einzuschließen.

2. Menstruationsscheibe nach Anspruch 1, wobei der innere Rand (110) und der äußere Rand (140) eine Vielzahl von Löchern (112) einschließen, die durch die Dicke des inneren Randes (110) bis zum äußeren Rand (140) angeordnet sind, um ein Saugprofil auf der Seite des äußeren Randes (140) der Menstruationsscheibe bereitzustellen, wenn diese vorübergehend in der Vaginalwand installiert ist.

3. Menstruationsscheibe nach Anspruch 1, wobei das Verhältnis zwischen der Randhöhe H1 und der Randdicke R1 etwa 1,83 beträgt.

4. Menstruationsscheibe nach Anspruch 2, wobei die Dicke R1 zwischen etwa 5,0 mm und etwa 6,0 mm beträgt.

5. Menstruationsscheibe nach Anspruch 3, wobei die Menstruationsscheibe einen Durchmesser D1 einschließt und die Menstruationsscheibe eine Scheibenhöhe H2 einschließt, wobei der Durchmesser D1 zwischen etwa 60 mm und 70 mm beträgt, die Randhöhe H1 zwischen etwa 9 mm und etwa 14 mm beträgt und die Scheibenhöhe H2 zwischen etwa 28 mm und etwa 35 mm beträgt.

6. Menstruationsscheibe nach Anspruch 1, wobei der zentrale Rastabschnitt ein wellenförmiges Querschnittprofil einschließt, um eine Minimierung der Saugkraft der Menstruationsscheibe bereitzustellen.

7. Menstruationsscheibe nach Anspruch 6, wobei der äußere Rand (140) eine texturierte Oberfläche (146) einschließt, die jegliche Dichtung mit der Vaginalwand unterbricht.

8. Menstruationsscheibe nach Anspruch 6, wobei die Breite U1 und die Tiefe U2 zwischen etwa 9 mm und etwa 15 mm betragen; der abgeschrägte Abschnitt eine Breite S1 zwischen etwa 0,5 mm und etwa 1,5 mm einschließt, wobei die Breite S1 größer ist als die Dicke des zentralen Rastabschnitts (130).

9. Menstruationsscheibe nach Anspruch 1, wobei der zentrale Rastabschnitt eine Rasthöhe C1 und eine Rastkapazität einschließt; wobei die Rastkapazität zwischen etwa 35 ml und etwa 50 ml beträgt, wenn der zentrale Rastabschnitt (130) im Vaginalkanal sitzt; und der zentrale Rastabschnitt (130) eine Rastdicke C2 zwischen etwa 0,1 mm und etwa 0,7 mm einschließt; wobei sich die Rastdicke C2 auf eine Randdicke C3 ausdehnt, wenn der zentrale Rastabschnitt (130) mit dem äußeren Rand (140) verbunden wird; wobei die Randdicke C3 zwischen etwa 0,8 mm und etwa 1,2 mm beträgt.

10. Menstruationsscheibe nach Anspruch 1, wobei der Griffabschnitt (142) mindestens zwei konzentrische Kreise umfasst.

11. Menstruationsscheibe nach Anspruch 10, wobei der äußere Rand (140) eine äußere Lippe (148) umfasst, die um die äußere Oberfläche des äußeren Randes (140) und zwischen der äußeren Oberfläche des zentralen Verschlusses angeordnet ist.

## Revendications

1. Disque menstruel (100) comprenant : un bord intérieur (110) relié fonctionnellement à une languette (120) et à une partie de rétention centrale (130), dans lequel le bord intérieur (110) et la languette (120) forment une ouverture généralement en forme de C ; la partie de rétention centrale (130) reliée fonctionnellement à un bord extérieur (140) comportant au moins une partie de préhension (142) et une partie supérieure incurvée (144a) ; dans lequel le bord extérieur (140) est un bord semi-rigide et souple conçu pour se replier vers l'intérieur vers un axe du disque menstruel pour passer d'une configuration ouverte à une configuration pliée ; l'axe allant d'une partie avant du disque menstruel à une partie arrière, l'au moins une partie de préhension (142) est positionnée perpendiculairement à cet axe pour permettre le repliement du disque vers l'intérieur de l'axe ; la partie de rétention centrale (130) est conçue pour contenir le flux menstruel en configuration ouverte comme en position pliée ; dans lequel la languette (120) comporte une rainure inférieure (124), une partie de bord (122) et une partie inclinée (126) se prolongeant vers le bas depuis le bord intérieur (110) jusqu'à la partie de rétention centrale (130) ; la partie de bord (122) s'emboîte de manière fonctionnelle avec la partie supérieure incurvée (144a) du bord extérieur (140) pour créer l'ouverture généralement en forme de C ; la rainure inférieure (124) comporte une largeur U1 et une profondeur U2, dans lequel la combinaison de la rainure inférieure (124), du bord intérieur (110) et d'une partie du bord extérieur (140) est dimensionnée pour permettre à un utilisateur de saisir deux ou plusieurs parmi la languette (120), le bord intérieur (110) et le bord extérieur (140) afin de retirer le disque menstruel du vagin ; dans lequel la largeur U1 et la profondeur U2 de la rainure inférieure (124) sont dimensionnées pour permettre à un utilisateur de localiser le disque menstruel à l'intérieur du vagin et dimensionnées pour que le doigt de l'utilisateur puisse saisir la languette (120), et dans lequel le haut de la languette (120) ne se prolonge pas au-dessus du haut du bord extérieur (140) et du bord intérieur (110) ;
**caractérisé en ce que** la partie inclinée (126) comporte une largeur S1 dimensionnée pour être supérieure à l'épaisseur de la partie de rétention centrale (130), afin de permettre une plus grande rigidité lorsque la languette (120) est retirée d'un axe du disque menstruel ; et **en ce que** le bord extérieur (140) comporte une épaisseur de bord R1 et une hauteur de bord H1, et que le rapport entre la hauteur de bord H1 et l'épaisseur de bord R1 est compris entre environ 0,9 et environ 2,0 ; la partie de bord (122) comporte un profil d'épaisseur triangulaire, ce qui permet au bord extérieur (140) de comporter une rigidité du semi-bord sur la partie supérieure et une épaisseur plus mince sur une partie inférieure de la partie de bord (122).

2. Disque menstruel selon la revendication 1, le bord intérieur (110) et le bord extérieur (140) comportent une pluralité de trous (112) disposés à travers l'épaisseur du bord intérieur (110) jusqu'au bord extérieur (140), afin de fournir un soulagement par succion sur le côté du bord extérieur (140) du disque menstruel lorsqu'il est temporairement installé dans la paroi vaginale.

3. Disque menstruel selon la revendication 1, dans lequel le rapport entre la hauteur de bord H1 et l'épaisseur de bord R1 est d'environ 1,83.

4. Disque menstruel selon la revendication 2, dans lequel l'épaisseur R1 est comprise entre environ 5,0 mm et environ 6,0 mm.

5. Disque menstruel selon la revendication 3, dans lequel le disque menstruel comporte un diamètre D1, et le disque menstruel comporte une hauteur de disque H2, dans lequel le diamètre D1 est compris entre environ 60 mm et 70 mm, la hauteur de bord H1 est comprise entre environ 9 mm et environ 14 mm, et la hauteur de disque H2 est comprise entre environ 28 mm et environ 35 mm.

6. Disque menstruel selon la revendication 1, dans lequel la partie de rétention centrale comporte un profil de section transversale ondulé afin de minimiser la force d'aspiration du disque menstruel.

7. Disque menstruel selon la revendication 6, dans lequel le bord extérieur (140) comporte une surface texturée (146) qui empêche toute étanchéité de se former avec la paroi vaginale.

8. Disque menstruel selon la revendication 6, dans lequel la largeur U1 et la profondeur U2 sont comprises entre environ 9 mm et environ 15 mm ; la partie inclinée comporte une largeur S1 comprise entre environ 0,5 mm et environ 1,5 mm, la largeur S1 est supérieure à une épaisseur de la partie de rétention centrale (130).

9. Disque menstruel selon la revendication 1, dans lequel la partie de rétention centrale comporte une hauteur de rétention C1 et une capacité de rétention ; dans lequel la capacité de rétention est comprise entre environ 35 ml et environ 50 ml lorsque la partie de rétention centrale (130) se trouve à l'intérieur du canal vaginal ; et la partie de rétention centrale (130) comporte une épaisseur de rétention C2 comprise entre environ 0,1 mm et environ 0,7 mm ; l'épaisseur de rétention C2 s'élargit à une épaisseur de bord C3 lorsque la partie de rétention centrale (130) se relie au bord extérieur (140) ; dans lequel l'épaisseur de bord C3 est comprise entre environ 0,8 mm et environ 1,2 mm.

10. Disque menstruel selon la revendication 1, dans lequel la partie de préhension (142) comprend au moins deux cercles concentriques.

11. Disque menstruel selon la revendication 10, dans lequel le bord extérieur (140) comprend une lèvre extérieure (148) disposée autour de la surface extérieure du bord extérieur (140) et entre la surface extérieure de la rétention centrale.
